# FASCICULE DE BREVET EUROPEEN

(11) **EP 1 768 623 B1**
(45) Date de publication et mention de la délivrance du brevet: **03.12.2008**
(21) Numéro de dépôt: 05791023.4
(22) Date de dépôt: 13.07.2005
(51) Int. Cl.: A61F 5/441

(54) **Filtre à évent de dégazage et poche de recueil de fluides corporels**
Filter mit Entgasungsöffnung und Beutel zum Sammeln von Körperflüssigkeiten
Filter provided with a degassing vent and pocket for collecting body fluids

(30) Priorité: 21.07.2004 FR 0408088
(43) Date de publication de la demande: 04.04.2007
(73) Titulaire: B.Braun Medical SAS, 92100 Boulogne-Billancourt (FR)
(72) Inventeur: MATHIEU, Florence, 42330 Saint Galmier (FR)
(74) Mandataire: Schmit, Christian Norbert Marie
(86) Numéro de dépôt international: PCT/FR2005/001813
(87) Numéro de publication internationale: WO 2006/021645

(56) Documents cités:
- EP-A- 0 294 257
- EP-A- 0 990 429
- US-A- 4 490 145
- US-A- 5 496 396
- US-A- 6 135 986

## Description

La présente invention concerne un filtre à évent de dégazage destiné à une poche de recueil de fluides corporels, ainsi qu'une telle poche.

L'invention concerne le problème de la désodorisation des gaz évacués par les stomisés, et elle s'applique essentiellement aux poches de stomie, notamment de colostomie et d'iléostomie.

Les fluides évacués par les stomisés contiennent des matières solides et liquides, mais aussi une certaine quantité de gaz. Si les poches de recueil n'étaient munies d'aucun dispositif d'évent, elles se gonfleraient et créeraient une gêne importante. Pour cette raison, on munit ces poches d'évents de dégazage comportant eux-mêmes un filtre ayant une matière adsorbante placée dans une enveloppe.

Une première caractéristique fondamentale que doivent posséder de tels filtres est qu'ils ne doivent pas permettre le passage du gaz autour de la matière adsorbante du filtre. Ce résultat est obtenu de diverses manières bien connues qui n'entrent pas dans le cadre de l'invention.

Une autre caractéristique importante est que la perte de charge dans le filtre ne doit pas être importante, afin que la poche ne gonfle pas.

Il existe de nombreux filtres qui donnent tout à fait satisfaction en l'absence de liquides. Cependant, en présence de liquides, même en petite quantité, les matières en suspension peuvent provoquer un bouchage du filtre, la matière adsorbante du filtre peut être saturée par les matières contenues dans le liquide, et le liquide peut remplir les interstices de la matière adsorbante et créer une perte de charge importante qui s'oppose au passage des gaz, si bien que le filtre n'est plus efficace pour la désodorisation des gaz. Le liquide peut même parfois traverser le filtre et souiller les vêtements.

L'invention concerne la protection de ces filtres à évent de dégazage contre l'action des liquides.

On sait déjà placer les filtres à la partie d'une poche qui doit se trouver à l'emplacement le plus haut lorsque la poche est portée. Cependant, comme les personnes qui portent les poches doivent pouvoir s'asseoir et se coucher, on ne peut pas compter sur cette simple disposition physique pour éviter le mouillage du filtre par les liquides.

Une telle solution est par exemple décrite dans le brevet européen EP-294 257 qui concerne un filtre à évent de dégazage disposé à une partie supérieure d'une poche et comprenant une colonne de matière adsorbante placée entre deux films de matière plastique et débouchant à deux bords opposés de la poche. L'entrée dans le filtre s'effectue par une simple perforation placée au centre. Le filtre se trouve à la partie supérieure de la poche lorsque celle-ci est portée par une personne debout.

Bien qu'un tel filtre donne satisfaction en général pour les poches de colostomie, il n'est pas toujours satisfaisant pour les poches d'iléostomie, car les matières évacuées sont beaucoup plus liquides.

Pour remédier à cet inconvénient, on a réalisé des poches de recueil ayant un filtre formant évent de dégazage et dans lesquelles le trajet des matières jusqu'à l'entrée du filtre est sinueux et comporte des parties destinées à tenir le liquide à distance de l'entrée du filtre, quelle que soit la position dans laquelle la poche est portée.

Plus précisément, le brevet EP-990 429 décrit un filtre disposé entre une chambre d'entrée et une chambre de sortie de gaz. La chambre de sortie de gaz communique avec l'atmosphère par un petit orifice et la chambre d'entrée communique avec l'intérieur de la poche de recueil par un passage sinueux. L'entrée du filtre est donc relativement à l'abri des liquides du fait de la protection procurée par la chambre d'entrée. Cependant, si malgré le trajet sinueux d'accès à la chambre d'entrée, du liquide parvient à celle-ci, par exemple parce que la personne qui porte la poche s'est couchée, le liquide piégé risque de pénétrer dans le filtre et de compromettre son fonctionnement.

On a donc essayé d'utiliser des matériaux hydrophobes pour empêcher des liquides aqueux d'entrer dans le filtre.

Par exemple, le brevet US-5 496 396 décrit un filtre dont la matière adsorbante contient un adsorbant mélangé à des particules de polytétrafluoréthylène, frittées et non frittées, destiné à empêcher le mouillage. Cependant, cette disposition n'empêche pas l'obstruction du filtre par le liquide, si bien que les gaz ne peuvent circuler que lorsqu'une pression suffisante pour chasser le liquide est appliquée.

Le brevet US-6 135 986 décrit un filtre désodorisant qui comprend un matériau poreux placé entre deux parois imperméables aux gaz et aux liquides ayant des ouvertures d'entrée et de sortie, et l'ouverture d'entrée est recouverte d'une membrane hydrophobe et oléophobe qui est elle-même couverte par une paroi supplémentaire qui délimite un espace pour le logement d'une mousse entre les parois. Selon ce dernier document, la membrane hydrophobe est séparée par une couche de mousse et une paroi supplémentaire du côté des fluides corporels.

L'invention concerne un perfectionnement des filtres à évent de dégazage permettant de remédier à l'inconvénient du contact du liquide avec la matière adsorbante. Selon l'invention, avant l'entrée dans la colonne de matière adsorbante, les gaz doivent traverser une feuille d'un matériau hydrophobe étanche aux liquides et perméable aux gaz dont la face opposée à l'entrée est directement accessible aux fluides corporels.

Plus précisément, l'invention concerne un filtre à évent de dégazage, destiné à une poche de recueil de fluides corporels, du type qui comprend une matière adsorbante sous forme d'une colonne allongée entourée, au moins sur sa plus grande partie disposée entre ses extrémités longitudinales, par un matériau étanche aux gaz et aux liquides, le filtre ayant au moins une entrée et au moins une sortie séparées par une longueur efficace de colonne de matière adsorbante, l'entrée au moins étant entièrement séparée du milieu extérieur par une feuille d'un matériau hydrophobe étanche aux liquides mais perméable aux gaz. Selon l'invention, la face de la feuille de matériau hydrophobe étanche aux liquides mais perméable aux gaz opposée à l'entrée est directement accessible aux fluides corporels, et la feuille du matériau hydrophobe étanche aux liquides et perméable aux gaz est à distance du matériau étanche aux gaz et aux liquides au moins à proximité d'une entrée.

Dans un mode de réalisation, le matériau étanche aux gaz et aux liquides est sous forme d'une feuille, et il recouvre toute la colonne, sauf à l'entrée et à la sortie.

Dans un mode de réalisation, une entrée au moins est formée par une perforation traversant la colonne et le matériau étanche aux gaz et aux liquides des deux côtés de la colonne.

De préférence, le matériau hydrophobe étanche aux liquides et perméable aux gaz est choisi parmi un non-tissé, une membrane et un stratifié formé d'un non-tissé et d'une membrane. Le non-tissé, la membrane et le stratifié sont avantageusement oléophiles. Le non-tissé et la membrane sont avantageusement formés de polypropylène ou de polyéthylène.

De préférence, le matériau étanche aux gaz et aux liquides est avantageusement une feuille d'une polyoléfine choisie parmi le polypropylène et le polyéthylène.

De préférence, le matériau hydrophobe étanche aux liquides et perméable aux gaz est maintenu à distance du matériau étanche aux gaz et aux liquides au moins à proximité d'une entrée par un dispositif d'entretoise.

Dans un mode de réalisation, le filtre comporte plusieurs entrées formées par des orifices, de nombre et dimensions divers, et/ou des perforations.

Dans un mode de réalisation, le filtre comporte une zone de pliage préférentiel. De préférence, cette zone de pliage préférentiel est constituée par une perforation de la colonne, de forme allongée en direction transversale à la longueur du filtre.

L'invention concerne aussi une poche de recueil de fluides corporels, du type qui est en communication avec l'atmosphère extérieure par un filtre à évent de dégazage ; selon l'invention, le filtre est tel qu'indiqué dans les paragraphes précédents, et une sortie du filtre est à distance des limites extérieures de la poche.

Dans le présent mémoire, on distingue un orifice, qui peut ne traverser qu'une épaisseur de matériau, par exemple une feuille de matériau imperméable aux gaz et aux liquides, d'une perforation, qui, appliquée au filtre, indique une traversée de plusieurs épaisseurs, telles que celles du matériau adsorbant et des deux feuilles de matériau imperméable aux gaz et aux liquides qui l'entourent.

D'autres caractéristiques et avantages de l'invention seront mieux compris à la lecture de la description qui suit d'exemples de réalisation, faite en référence aux dessins annexés sur lesquels :
la figure 1 est une vue en perspective d'un exemple simple de filtre connu ;
la figure 2 est une coupe d'une extrémité d'entrée du filtre de la figure 1 ;
la figure 3 illustre le principe de mise en oeuvre de l'invention ;
la figure 4 est une vue en perspective d'un mode de réalisation de l'invention ;
la figure 5 est une coupe d'une partie du filtre de la figure 4 ;
la figure 6 est une coupe analogue à la figure 5 d'une partie d'un filtre selon un autre mode de réalisation de l'invention ;
la figure 7 représente un exemple d'entrée de filtre ; et
la figure 8 est une coupe schématique d'une partie de poche munie d'un filtre selon l'invention.

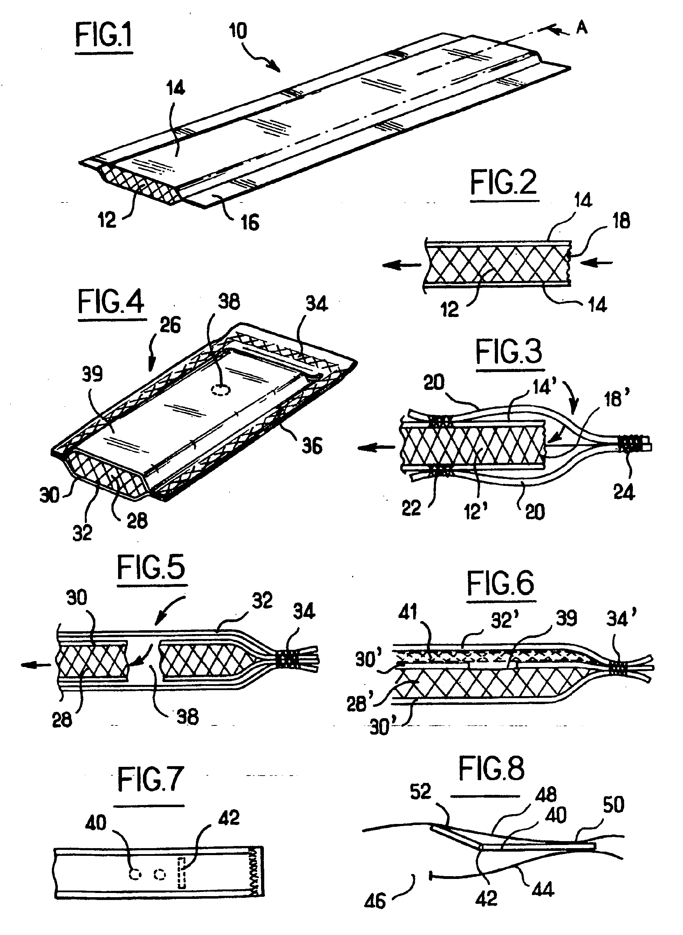

La figure 1 représente un exemple de filtre connu 10. Une colonne 12 de matière adsorbante, par exemple de charbon actif, est placée entre deux feuilles étanches aux liquides et aux gaz 14, soudées sur leurs bords 16. Ce filtre 10 provoque une certaine compression de la colonne 12 du fait de l'élasticité des feuilles 14, de sorte qu'il ne se forme aucun chemin préférentiel de passage de gaz le long de la colonne 12 à l'intérieur des feuilles 14 entre une entrée 18 et une sortie de gaz. On note ainsi sur la figure 2 que, lorsqu'il a pénétré à l'entrée 18, le gaz circule dans la matière adsorbante de la colonne 12, entre les feuilles 14 qui restent bien appliquées contre la matière adsorbante. On ne décrit pas plus en détail la coopération entre la colonne et les feuilles qui empêche la formation d'un trajet préférentiel de gaz le long des feuilles 14, car il existe d'autres moyens pour obtenir ce résultat, et ces moyens ont fait l'objet d'un certain nombre de brevets.

Lorsqu'un tel filtre est placé dans une poche d'iléostomie par exemple, qui reçoit des excréments relativement fluides, des matières aqueuses peuvent venir au contact de l'entrée 18 de la colonne de filtre. L'eau peut pénétrer dans la colonne et en remplir les interstices. Une obstruction peut être due d'une part aux matières en suspension qui bouchent les interstices ou pores, et d'autre part à l'eau qui remplit les pores de sorte qu'il faut une pression élevée pour que les gaz sortent, si bien que la poche gonfle. Dans un cas extrême, le liquide peut même traverser le filtre et fuir à l'extérieur de la poche.

Selon l'invention, ce problème est résolu par disposition d'une feuille d'un matériau étanche aux liquides et perméable aux gaz entre l'entrée et les fluides corporels.

La figure 3 représente schématiquement le principe de l'invention. Selon l'invention, l'entrée 18' d'une colonne 12' de charbon actif maintenue entre deux feuilles de matière plastique étanches aux gaz et aux liquides 14' est protégée des fluides corporels par une feuille 20 d'un matériau hydrophobe étanche aux liquides mais perméable aux gaz, fixée par soudage en 22 aux feuilles 14' du filtre et en 24 sur elle-même ; ainsi, tout passage direct entre l'extérieur de la feuille et l'entrée 18' est interdit. Pour pouvoir circuler dans la colonne 12', les gaz doivent avoir traversé la feuille 20 du matériau hydrophobe perméable aux gaz.

Les figures 4 et 5 représentent un mode de réalisation de l'invention. Dans ce mode de réalisation, l'entrée des gaz ne s'effectue pas à l'extrémité de la colonne 28, mais par les orifices d'une perforation 38 formée dans la colonne et les feuilles 30 de matière plastique étanches aux liquides et aux gaz qui l'entourent.

A l'extrémité du filtre, les feuilles étanches aux liquides et aux gaz 30 et les feuilles hydrophobes 32 sont soudées en 34 et ferment de manière étanche l'extrémité. De même, les côtés sont fermés de manière étanche par des soudures 36. A cet effet, les matériaux de la feuille étanche aux liquides et aux gaz 30 et de la feuille hydrophobe étanche aux liquides mais perméable aux gaz 32 sont de préférence compatibles afin qu'ils permettent le soudage.

Bien entendu, à un ou plusieurs emplacements de la longueur, les feuilles sont aussi soudées mutuellement afin qu'il ne puisse pas se former un trajet préférentiel vers la sortie, entre les deux feuilles. En général, à cet effet, le filtre est soudé à la poche dans la région 39 et, dans cette région, les deux feuilles sont aussi soudées de sorte que les gaz qui traversent la feuille hydrophobe 32 ne peuvent pas parvenir directement à la sortie.

La quantité de gaz qui peut être évacuée dépend donc d'une part de la perméabilité aux gaz concernés de la feuille 32 et d'autre part de la surface des entrées, par exemple des orifices de la perforation 38.

On peut utiliser une membrane de polyéthylène ultra-haute densité. Par exemple, on connaît une membrane de polyéthylène très poreuse disponible dans le commerce sous la dénomination "Solupor", ayant une porosité de 85% et une perméabilité à l'air très élevée. Grâce à cette perméabilité élevée, une telle membrane peut suffire même si elle n'est présente que d'un côté du filtre.

On peut aussi utiliser un non-tissé de polyéthylène ou de polypropylène stratifié à une membrane mince de polyéthylène ou de polypropylène. Par exemple, on connaît un non-tissé de polypropylène stratifié à une membrane mince de polypropylène disponible dans le commerce sous la dénomination "Linopore". Dans ce dernier cas, pour que le soudage de la feuille hydrophobe à une feuille étanche aux gaz et aux liquides soit efficace, il est préférable que la membrane soit tournée vers la feuille étanche aux gaz et aux liquides et que le non-tissé soit tourné du côté opposé.

Plus la feuille hydrophobe est perméable aux gaz, plus les orifices des entrées peuvent être petits. Il suffit le plus souvent d'une seule entrée de quelques millimètres carrés de section. Si la perméabilité de la feuille hydrophobe est insuffisante, il est possible d'accroître la section d'orifice par laquelle le gaz peut pénétrer. Ainsi, on peut soit augmenter la surface utile de la feuille perméable aux gaz, comme décrit dans la suite en référence à la figure 6, soit utiliser plusieurs orifices ou perforations, comme indiqué sur la figure 7 qui représente un exemple dans lequel deux orifices circulaires 40 et une perforation allongée transversalement 42 sont formés dans la colonne.

Grâce au grand pouvoir désodorisant de la colonne adsorbante, la longueur de colonne comprise entre l'entrée le plus proche de la sortie et cette dernière peut être réduite, par exemple à un ou deux centimètres seulement. Ainsi, sur la figure 7, la partie ayant les orifices d'entrée 40 et la perforation 42 peut avoir une longueur de 2 cm, et les parties placées de part et d'autre peuvent aussi avoir chacune une longueur de 2 cm. Un tel filtre est avantageux, par exemple dans la réalisation décrite dans la suite du présent mémoire en référence à la figure 8. Les orifices 40 peuvent aussi être formés par des perforations qui traversent la colonne.

En outre, bien qu'on ait indiqué la présence d'orifices d'entrée peu nombreux et relativement grands, il est possible d'obtenir une surface importante d'entrée avec un nombre grand ou très grand d'orifices petits ou très petits, tels que des pores.

Bien qu'on ait indiqué que les feuilles étaient soudées, elles peuvent évidemment être fixées d'une autre manière, par exemple par collage.

La figure 6 est une coupe analogue à la figure 5 d'une partie d'un filtre selon un autre mode de réalisation de l'invention. Sur cette figure, une colonne 28' de matériau adsorbant est entourée de deux feuilles 30' de matériau imperméable aux gaz et aux liquides. Une seule de ces deux feuilles a un orifice 39 qui dégage le matériau adsorbant. Une feuille 32' de matériau hydrophobe étanche aux liquides mais perméable aux gaz est disposée vers l'extérieur de la feuille 30' ayant l'orifice 39. Entre ces deux feuilles 30' et 32' est disposée une couche 41 d'une mousse très perméable aux gaz, constituant un dispositif d'entretoise et dont le rôle est d'écarter la feuille 32' de la feuille 30', tout en permettant la circulation des gaz parallèlement à la surface des feuilles 30' et 32'.

Alors que, dans le mode de réalisation de la figure 5, seule la partie de feuille 32 voisine de la perforation 38 est réellement utile pour la traversée des gaz, dans le mode de réalisation de la figure 6, les gaz peuvent traverser la feuille 32' sur une très grande surface avant de parvenir à l'orifice 39 en se déplaçant dans la couche de mousse 41 qui est très perméable. Il est ainsi possible d'utiliser une feuille de matériau hydrophobe étanche aux liquides mais perméable aux gaz de perméabilité modérée, puisqu'elle est active sur une grande surface.

La couche 41 de mousse très perméable aux gaz qui permet la circulation des gaz est avantageusement une mousse à cellules ouvertes, par exemple de polyuréthanne ou de polyéthylène. Elle peut être collée à l'une au moins des feuilles qui lui sont adjacentes, par exemple par un adhésif thermofusible. Elle peut être renforcée par une grille de polyamide, analogue à un tulle. L'orifice ou les orifices tels que 39 peuvent aussi être formés dans la mousse.

Bien qu'on ait décrit une couche de mousse pour écarter les deux feuilles 30' et 32' l'une de l'autre, tout autre dispositif d'entretoise équivalent peut être utilisé, dans la mesure où il permet une augmentation de la surface réellement utile de la feuille hydrophobe perméable aux gaz.

Bien qu'on ait décrit la disposition de la couche de mousse d'un seul côté du filtre, elle peut être placée des deux côtés de celui-ci.

La figure 7 représente une caractéristique supplémentaire. Lorsque le filtre est fixé dans une poche, il est normalement soudé aux parois de la poche à proximité de la sortie, et il est aussi le plus souvent fixé à un autre emplacement. Etant donné que le filtre a une certaine épaisseur et donc une certaine rigidité, il peut être avantageux de permettre son pliage à un emplacement en amont d'au moins une entrée pour éviter que le pliage ne provoque une perte de charge trop importante, ou même n'obstrue complètement le filtre en empêchant tout dégazage. Cette caractéristique de pliage préférentiel peut être avantageusement obtenue par formation de perforations allongées telles que la perforation 42. Ainsi, cette perforation permet au filtre de se déplacer lors de la déformation de la poche, pour que la feuille perméable reste en grande partie à distance des parois de la poche pour permettre l'entrée des gaz. De préférence, chaque perforation, telle que 42, est suffisamment étroite pour que les deux feuilles hydrophobes placées des deux côtés du filtre ne puissent pas venir en contact mutuel, car la pénétration des gaz deviendrait nulle dans les parties de contact.

On pourrait craindre que les parois imperméables de la poche associée et les liquides contenus par celle-ci ne soient au contact du film hydrophobe et n'empêchent ainsi la pénétration du gaz par les entrées. Il est donc souhaitable que le filtre ait tendance à s'écarter des parois de la poche et des matières que celle-ci peut contenir.

La figure 8 représente un mode de réalisation de filtre incorporé à une poche qui résout le problème ainsi évoqué. Dans cet exemple, un filtre tel que représenté sur la figure 7 est disposé entre une paroi interne 44 de la poche (du côté de la peau du patient) ayant un orifice 46 pour la stomie, et une paroi externe 48. Il est soudé en 50 aux deux parois afin que sa sortie débouche à l'atmosphère, mais de préférence en-deçà du bord des parois 44 et 48.

Un emplacement proche de l'extrémité interne du filtre est fixée à la paroi externe, par exemple par un point de soudure ou de colle 52. Comme le filtre possède une perforation 42 qui constitue un emplacement de pliage préférentiel, lorsque les parois 44 et 48 de la poche s'écartent l'une de l'autre, l'extrémité du filtre suit la paroi externe 48 en pliant le filtre au niveau de la perforation allongée 42.

On note ainsi que, dans la position représentée sur la figure 8, qui correspond au cas d'un patient couché, le filtre s'écarte d'une part des matières retenues dans la poche (non représentées), et d'autre part de la paroi externe 48 elle-même, si bien qu'une surface importante des entrées du filtre est disponible pour le passage des gaz, même en présence d'une grande quantité de liquides.

Pour favoriser cette déformation, il est possible de rigidifier les parties qui ne doivent pas se plier, par exemple par renforcement local des soudures latérales du filtre (augmentation d'épaisseur, insertion d'une tige de renfort, addition d'une couche continue ou non au matériau étanche aux gaz et aux liquides d'un côté du filtre, etc.).

On peut ainsi maîtriser le comportement du filtre d'une part en créant des zones de pliage préférentiel, telles que la perforation allongée 42, et d'autre part des zones de renforcement préférentiel du filtre.

On a indiqué que la colonne adsorbante était formée de charbon actif. Bien entendu, d'autres matières adsorbantes bien connues peuvent être utilisées, leurs caractéristiques essentielles étant de fixer l'hydrogène sulfuré.

Il est inutile que le filtre soit placé dans une poche du type décrit dans le brevet EP-990 429, c'est-à-dire entre une chambre d'entrée et une chambre de sortie de gaz, la chambre d'entrée étant reliée au reste de la poche par un trajet sinueux, car il suffit que le filtre ne soit pas toujours au contact du liquide au niveau des entrées. Il est donc préférable que le filtre soit disposé à un emplacement de la poche qui n'est pas constamment rempli des liquides corporels.

Bien entendu, diverses modifications peuvent être apportées par l'homme de l'art aux filtres et poches qui viennent d'être décrits uniquement à titre d'exemple non limitatif sans sortir du cadre de l'invention.

## Revendications

1. Filtre à évent de dégazage, destiné à une poche de recueil de fluides corporels, du type qui comprend une matière adsorbante sous forme d'une colonne allongée (28) entourée, au moins sur sa plus grande partie disposée entre ses extrémités longitudinales, par un matériau étanche aux gaz et aux liquides (30), le filtre ayant au moins une entrée (38, 40, 42) et au moins une sortie séparées par une longueur efficace de colonne (28) de matière adsorbante, l'entrée (38, 40, 42) au moins étant entièrement séparée du milieu extérieur par une feuille d'un matériau hydrophobe étanche aux liquides mais perméable aux gaz (32) **caractérisé en ce que** la face de la feuille de matériau hydrophobe étanche aux liquides mais perméable aux gaz (32) opposée à l'entrée (38, 40, 42) est directement accessible aux fluides corporels, et la feuille du matériau hydrophobe étanche aux liquides et perméable aux gaz (32) est à distance du matériau étanche aux gaz et aux liquides (30) au moins à proximité d'une entrée.

2. Filtre selon la revendication 1, **caractérisé en ce que** le matériau étanche aux gaz et aux liquides (30) est sous forme d'une feuille, et il recouvre toute la colonne (28), sauf à l'entrée (38, 40, 42) et à la sortie.

3. Filtre selon l'une des revendications 1 et 2, **caractérisé en ce qu'**une entrée au moins est formée par une perforation (42) traversant la colonne (28) et le matériau étanche aux gaz et aux liquides (30) des deux côtés de la colonne.

4. Filtre selon l'une quelconque des revendications précédentes, **caractérisé en ce que** le matériau hydrophobe étanche aux liquides et perméable aux gaz (32) est choisi parmi un non-tissé et un stratifié formé d'un non-tissé et d'une membrane.

5. Filtre selon l'une quelconque des revendications précédentes, **caractérisé en ce que** le matériau hydrophobe étanche aux liquides et perméable aux gaz (32') est maintenu à distance du matériau étanche aux gaz et aux liquides (30') par un dispositif d'entretoise (41).

6. Filtre selon l'une quelconque des revendications précédentes, **caractérisé en ce que** l'entrée comprend un grand nombre de petits orifices.

7. Filtre selon l'une quelconque des revendications précédentes, **caractérisé en ce que** le filtre comporte plusieurs entrées (38, 40, 42) formées par des perforations de la colonne et du matériau étanche aux gaz et aux liquides (30).

8. Filtre selon l'une quelconque des revendications précédentes, **caractérisé en ce qu'**il comporte une zone de pliage préférentiel (42).

9. Filtre selon les revendications 7 et 8 prises ensembles, **caractérisé en ce qu'**une zone de pliage préférentiel (42) est constituée par une perforation (42) de forme allongée en direction transversale à la longueur du filtre.

10. Poche de recueil de fluides corporels, du type qui est en communication avec l'atmosphère extérieure par un filtre à évent de dégazage, **caractérisée en ce que** le filtre est selon l'une quelconque des revendications précédentes, et une sortie du filtre est à distance des limites extérieures de la poche.

## Claims

1. A filter provided with a degassing vent, intended for a bag for collecting body fluids, of the type which comprises an adsorbent material in the form of an elongate column (28) surrounded, at least over it largest part disposed between its longitudinal ends, by a gas-tight and liquid-tight material (30), the filter having at least one inlet (38, 40, 42) and at least one outlet which are separated by an effective length of column (28) of adsorbent material, the inlet (38, 40, 42) at least being entirely separated from the external ambience by a sheet of liquid-tight but gas-permeable hydrophobic material (32), **characterized in that** the face of the sheet of liquid-tight but gas-permeable hydrophobic material (32) opposite to the inlet (38, 40, 42) is directly accessible to the body fluids, and the sheet of liquid-tight and gas-permeable hydrophobic material (32) is at a distance from the gas-tight and liquid-tight material (30) at least in proximity to an inlet.

2. A filter according to claim 1, **characterized in that** the gas-tight and liquid-tight material (30) is in the form of a sheet, and that it covers the whole column (28), except at the inlet (38, 40, 42) and at the outlet.

3. A filter according to either claim 1 or claim 2, **characterized in that** one inlet at least is formed by a perforation (42) passing through the column (28) and the gas-tight and liquid-tight material (30) on both sides of the column.

4. A filter according to any one of the preceding claims, **characterized in that** the liquid-tight and gas-permeable hydrophobic material (32) is selected from among a nonwoven and a laminate formed of a nonwoven and of a membrane.

5. A filter according to any one of the preceding claims, **characterized in that** the liquid-tight and gas-permeable hydrophobic material (32') is maintained at a distance from the gas-tight and liquid-tight material (30') by a spacer device (41).

6. A filter according to any one of the preceding claims, **characterized in that** the inlet comprises a large number of small orifices.

7. A filter according to any one of the preceding claims, **characterized in that** the filter comprises a plurality of inlets (38, 40, 42) formed by perforations of the column and of the gas-tight and liquid-tight material (30).

8. A filter according to any one of the preceding claims, **characterized in that** it comprises a preferential folding zone (42).

9. A filter according to claims 7 and 8 taken together, **characterized in that** a preferential folding zone (42) is constituted by a perforation (42) of elongate shape in a direction transverse to the length of the filter.

10. A bag for collecting body fluids, of the type which is in communication with the external atmosphere through a filter provided with a degassing vent, **characterized in that** the filter is according to any one of the preceding claims, and an outlet of the filter is at a distance from the outer limits of the bag.

## Patentansprüche

1. Filter mit Entgasungsöffnung, der für einen Beutel zum Sammeln von Körperflüssigkeiten bestimmt ist, von dem Typ, der ein adsorbierendes Material in Form einer länglichen Säule (28) aufweist, die mindestens an ihrem größten zwischen ihren Längsenden liegenden Teil von einem gegen Gas und Flüssigkeiten dichten Material (30) umgeben ist, wobei der Filter mindestens einen Eingang (38, 40, 42) und mindestens einen Ausgang hat, die durch eine effektive Länge der Säule (28) aus adsorbierendem Material getrennt sind, wobei mindestens der Eingang (38, 40, 42) völlig von der äußeren Umgebung durch eine wasserabstoßende Folie getrennt ist, die gegen Flüssigkeiten dicht aber gegen Gase (32) durchlässig ist, **dadurch gekennzeichnet, dass** die dem Eingang (38, 40, 42) gegenüberliegende Seite der Folie aus wasserabweisendem Material, das gegen Flüssigkeiten dicht aber gegen Gase (32) durchlässig ist, direkt für Körperflüssigkeiten zugänglich ist und sich die Folie aus wasserabweisendem Material, das gegen Flüssigkeiten dicht und gegen Gase (32) durchlässig ist, mindestens in der Nähe eines Eingangs im Abstand von dem gegen Gase und Flüssigkeiten (30) dichten Material befindet.

2. Filter nach Anspruch 1, **dadurch gekennzeichnet, dass** sich das gegen Gase und Flüssigkeiten dichte Material (30) in der Form einer Folie darstellt und die ganze Säule (28) außer an dem Eingang (38, 40, 42) und dem Ausgang bedeckt.

3. Filter nach einem der Ansprüche 1 und 2, **dadurch gekennzeichnet, dass** mindestens ein Eingang durch eine Perforation (42) gebildet ist, welche die Säule (28) und das gegen Gase und Flüssigkeiten dichte Material (30) auf beiden Seiten der Säule durchquert.

4. Filter nach einem der vorhergehenden Ansprüche, **dadurch gekennzeichnet, dass** das wasserabweisende, gegen Flüssigkeiten dichte und gegen Gase (32) durchlässige Material unter einem nicht gewebten Material und einem geschichteten Material gewählt ist, das aus einem nicht gewebten Material und einer Membran gebildet ist.

5. Filter nach einem der vorhergehenden Ansprüche, **dadurch gekennzeichnet, dass** das gegen Flüssigkeiten dichte und gegen Gase durchlässige Material (32') durch eine Zwischenstückvorrichtung 41 von dem gegen Gase und Flüssigkeiten dichten Material (30') im Abstand gehalten wird.

6. Filter nach einem der vorhergehenden Ansprüche, **dadurch gekennzeichnet, dass** der Eingang eine große Anzahl von kleinen Öffnungen aufweist.

7. Filter nach einem der vorhergehenden Ansprüche, **dadurch gekennzeichnet, dass** der Filter mehrere Eingänge (38, 40, 42) aufweist, die durch Perforationen der Säule und des gegen Gase und Flüssigkeiten dichten Materials (30) gebildet sind.

8. Filter nach einem der vorhergehenden Ansprüche, **dadurch gekennzeichnet, dass** er eine Vorzugsfaltzone (42) aufweist.

9. Filter nach den Ansprüchen 7 und 8 gemeinsam, **dadurch gekennzeichnet, dass** eine Vorzugsfaltzone (42) durch eine Perforation (42) von länglicher Form in Querrichtung zu der Länge des Filters gebildet ist.

10. Beutel zum Sammeln von Körperflüssigkeiten von dem Typ, der mit der Außenatmosphäre durch einen Filter mit Entgasungsöffnung in Verbindung steht, **dadurch gekennzeichnet, dass** der Filter nach einem der vorhergehenden Ansprüche gebildet ist, und sich ein Ausgang des Filters im Abstand von den äußeren Grenzen des Beutels befindet.
